# EUROPEAN PATENT APPLICATION

(11) **EP 0 643 077 A1**
(43) Date of publication of application: **15.03.1995**
(21) Application number: 94114434.7
(22) Date of filing: 14.09.1994
(51) Int. Cl.: C07K 16/28, C12P 21/08, C12N 5/20, A61K 39/395, G01N 33/577

(54) **Monoclonal antibody against B70 molecule**

(30) Priority: 14.09.1993 JP 228540/93
(71) Applicant: SUMITOMO ELECTRIC INDUSTRIES, LTD, Osaka-shi, Osaka-fu 541 (JP)
(72) Inventor: Azuma, Miyuki, Tokyo-to (JP); Ito, Daisuke, Tokyo-to (JP); Yagita, Hideo, Tokyo-to (JP); Okumura, Ko, Chiba-shi, Chiba-ken (JP)
(74) Representative: Hansen, Bernd, Dr. Dipl.-Chem.

(57) **Abstract**

A monoclonal antibody against a molecule which can bind to CD28, CTLA-4 and is located on the activated B cell surface, i.e. B70, and its functional fragments are provided. A hybridoma which can produce the monoclonal antibody and a method for the production of a monoclonal antibody by using said hybridoma are also provided. The monoclonal antibody of this invention is useful for the study of the analysis of T-B cell interaction and for the clinical treatment including immunotherapy in transplantation, diagnosis of autoimmune diseases or the like.

## Description

### FIELD OF THE INVENTION

This invention relates to a monoclonal antibody and its functional fragments, more particularly a monoclonal antibody which reacts specifically with B70 which is expressed on the surface of an activated B cell membrane, or its functional fragments, a method for producing the monoclonal antibody, and a hybridoma being capable of producing the monoclonal antibody. The monoclonal antibody of this invention is useful for the immunotherapy or diagnosis for rejection in transplantation, autoimmune diseases, allergic diseases, cancers, etc. The "functional fragment" of the monoclonal antibody means fragments of the antibody which have an antigen-antibody reactivity, and includes specifically F(ab')₂, Fab', Fab, Fv, recombinant Fv and single chain Fv.

### BACKGROUND OF THE INVENTION

Immune response happens based on the mechanism of complicated and interaction of various kinds of cells and regulatory factors. It is the same in the case of the interaction of T cell and B cell. The main immune response of T cell and B cell is recognized via the binding of an antigen/MHC class II complex expressed on the surface of B cell with a T cell receptor (TCR)/CD3 complex on the surface of T cell. However, the TCR and the antigen/MHC complex has not high affinity, and the adhesion of between T cell-B cell is effected by using various kinds of molecules and submolecules, for example, LFA-1 and ICAM-1, ICAM-2, ICAM-3; CD2 and LFA-3; CD48; CD4 or CD 8 and MHC class I, II; etc., which are expressed in T cell and B cell, respectively. These molecules exhibit widely their functions not only in the interaction of T-B cells but also in the adhesion between leukocytes and target cells, tissues and blood tube endothelium, and the like. It is very important to clarify the functions of these adhesion molecules not only for the study of immunology but also for clinical study.

It has recently been clarified that a molecule which has been considered to be a differential antigen specific to T cell or B cell, as well as the above molecules, play an important role in the interaction of T-B cells. Those cells include CD28-B7/BB1 system. CD28 is a homodimer having a 90 kd which can be expressed in the peripheral T cell surface membrane, and B7/BB1 are a membrane molecule of an immunoglobulin superfamily, which are expressed in an activated B cell or macrophage which are antigen-presenting cells, dendric cells, and the like. The mechanism of the reaction between T-B cells, to which adhesion molecules participate, is illustrated below.
(1) An antigen peptide presented by MHC on the antigen-presenting cells including B cell is recognized by T cell via TCR, and then the first signal is given within the cells, by which T cell is activated and expression of CD28 is increased.
(2) On the other hand, B cell is activated by crosslinking of IL-4 or an antigen receptor and thereby B7/BB1 are expressed on cell surface. The expression is enhanced by a signal via MHC class II. In vivo, B cell recognizes the antigen with the antigen receptor on the surface thereof, and thereby the signal is transduced within the cell and then the expression of B7/BB1 is derived.
(3) Further, an antigen is presented by MHC of the surface of B cell. T cell recognizes it via TCR, and thereby a signal is transduced from MHC into B cell, and the expression of B7 will be increased.
(4) CD28 on T cell binds to B7/BB1 on B cell, and then, the second activated signal is transduced to T cell, and mRNA of cytokine is stabilized and thereby the production of IL-2, GM-CSF, IFN-γ, TNF-α, etc. is increased and further protein tyrosine is phosphorylated within the cells.
(5) Besides, CTLA-4 having high homology with CD28 is also expressed on the activated T cell. The CTLA-4 is expressed only in several percentages of CD28, but is a membrane molecule having a high affinity with B7/BB1 and is considered to be able to transfer the T cell-activating signal in cooperation with CD28. The T cell-activation via CD28 is different from the initiation via CD3/TCR but shows resistance to immunosuppression. Accordingly, when only a signal from TCR is given but no signal via CD28 is transferred, T cell will be suffered from clonal anergy.

The above will be explained in more detail as to the exhibition of cytotoxicity via CD28/B7 interaction in T cell. It is known that CD28-positive human cell strain YT2C2 kills a human B cell strain JY in vitro. This reaction is blocked by an anti-CD28 antibody or an anti-B7 antibody, which means that the interaction of CD28/B7 plays an important role in the mechanism of T cell dependent cytotoxicity.

However, the anti-CD28 antibody can blocked completely the cytotoxicity, but the anti-B7 antibody can only partially block it. This suggests that the activated B cell surface contains a molecule being capable of binding to CD28 and CTLA-4 on the T cell surface other than B7/BB1 and further that such a molecule plays also an important role in the T-B cells interaction.

Accordingly, in order to study further the T-B cells interaction to find a means useful for therapy of diseases, it will be necessary to clarify the function of the molecule which provides T cell a co-stimulatory signal together with B7/BB1.

For achieving the above purpose, it is important to obtain a monoclonal antibody against said substance. The present inventors have succeeded to obtain a monoclonal antibody against a molecule which can bind to CD28/CTLA-4 on T cell surface, other than B7/BB1 expressed on the activated B cell surface, and the new molecule is designated as "B70".

That is, this invention provides a monoclonal antibody against a molecule which can bind to CD28, CTLA-4 and is located on the activated B cell surface, i.e. B70, and its functional fragments. Moreover, this invention provides also a hybridoma being capable of producing the monoclonal antibody and a method for the production of a monoclonal antibody by using said hybridoma.

The monoclonal antibody of this invention is useful for the study of the analysis of T-B cell interaction, i.e. the role of CD28 and CTLA-4 expressed on each cell surface, B7/BB1, and B70, and further for actual use thereof in clinic. Moreover, the antibody of this invention can be used for inhibition of the binding of T-B cells and thereby making T cell tolerant to the antigen. Thus, the antibody of this invention is useful for the immunotherapy in transplantation. Likewise, the antibody can be used for diagnosis of autoimmune diseases via detection of cells which express B70. Moreover, owing to its activity for inhibiting the production of autoantibody by B cell, it can be used for the treatment of autoimmune diseases.

The monoclonal antibody of this invention can be produced by a well known method in this field as follows.

That is, a rodent is immunized with an activated B cell strain, and the spleen cell is fused with a mouse myeloma cell, and the produced hybridoma is selected by an indication of the inhibitory effect against the cytotoxicity of T cell to B cell strain, and finally, an antibody being capable of completely inhibiting the reaction of CD28-B70 system is selected, and then the antibody is isolated and purified from the culture supernatant of the antibody-producing hybridoma or ascites. In more detail, the method comprises by the following steps:
(i) immunizing a rodent with a human B cell strain (JY),
(ii) taking out the spleen from the immunized rodent and preparing a suspension of spleen cells,
(iii) mixing the spleen cell suspension with mouse myeloma cells in the presence of a fusion-promoting agent to effect the fusion of both cells,
(iv) culturing the fused cells in a medium which does not support unfused myeloma cells to select a hybridoma formed by fusion of the antibody-producing cells and myeloma cells,
(v) confirming the presence of anti-B70 antibody in the supernatant of each culture well containing the hybridoma with the indication of the complete inhibitory activity of CD28-positive cell strain YT2C2 (NK cell) mediated cytotoxicity against JY (B cell strain),
(vi) selecting the hybridoma being capable of producing the desired antibody, and obtaining a single clone by a limiting dilution method, and
(vii) recovering the antibody from the culture supernatant of the hybridoma of single clone.

Any rodent host animals may be used in this invention but Balb/c mouse is preferable, and the mouse myeloma cell is preferably p3U1. Besides, the medium which does not support the unfused myeloma cell used in the above step (iv) includes, for example, HAT medium.

The monoclonal antibody of this invention can be produced from the hybridoma by a well known method in this field.

The antibody of this invention has the following characteristics.
1) It can immunochemically bind to B70, i.e. a protein having a molecular weight of about 70 kd which is expressed specifically in an activated B cell and peripheral blood monocyte cell but does not react with B7/BB1.
2) The B70 to be reacted with the antibody is a protein which can bind to CD28 or CTLA-4 which are expressed on the surface of T cell, etc. and participates to the adhesion of T-B cells.
3) It shows an inhibitory activity against the binding of B70 with CD28 or CTLA-4.
4) It can inhibit the CD28-dependent T cell cytotoxicity.
5) It can inhibit mixed lymphocyte response.

As mentioned hereinabove, the monoclonal antibody of this invention is useful not only for the immunochemical study but also immunotherapy or diagnosis in various diseases. A person skilled in the art will readily understand that in order to achieve the objects, it is not necessarily required to used whole of the antibody molecule, but a fragment of the molecule can be used as far as it shows an activity, and occasionally, the fragment is more preferable. Accordingly, this invention includes also the functional fragments of the anti-B70 monoclonal antibody. The functional fragment means as mentioned hereinabove.

### Brief Description of Drawings:

Fig. 1 is a graph showing the inhibitory activity of IT-2 antibody against cytotoxicity.

Fig. 2 shows inhibitory effect of IT-2 against the reaction of JY and CTLA-4-Ig determined with FACS.

Fig. 3 shows a schematic drawing of an electrophoretic pattern obtained by immunoprecipitation of B70 molecule from JY by using IT-2 antibody.

Fig. 4 shows a graph of expression amount of B70 molecule with IT-2 antibody in human T cell strain determined with FACS.

Fig. 5 shows a graph of expression amount of B70 molecule with IT-2 antibody in human monocytic cell strain determined with FACS.

Fig. 6 shows a graph of expression amount of B70 molecule with IT-2 antibody in human B cell strain determined with FACS.

Fig. 7 shows a graph of expression amount of B70 molecule with IT-2 antibody in normal human peripheral blood lymphocytes and tonsillar lymphocytes of a patient suffered from chronic tonsillitis determined with FACS.

The following Examples are provided to further illustrate the present invention and are not to be construed as limiting thereof.

### Example 1

### (1) Immunization

A human EB virus transformed B cell strain: JY (available from Juntendo University, School of Medicine, the Department of Immunology) in the form of a physiologically saline solution (containing in 1 x 10⁷ cells/ml) was intraperitoneally injected to female BALB/c mouse (5 weeks age). After one week, the animal was subjected to the same treatment in every week, totally four times to immunize the animal.

### (2) Fusion

Three days after the final immunization treatment, the spleen was taken out from the mouse. The spleen thus taken out was cut and filtered with a mesh and then suspended into RPMI1640 medium (manufactured by Nissui) to give spleen cells (1 x 10⁸ cells). The spleen cells were mixed with a mouse-origin 8-azaguanine resistant strain (hypoxanthine guanine phosphoribosyl transferase defective strain): P3U1 (ATCC CRL 1597) (2 x 10⁷ cells) in a ratio of about 5 : 1, and the mixture was centrifuged (1500 rpm, 5 minutes). To the-thus obtained pellets of the cells was added a 50% polyethylene glycol 4000 (Merck)/RPMI1640 solution (2 ml) with stirring in a water bath at 37°C over a period of one minute, by which the fusion of cells was effected. After the cell fusion, a large amount (about 40 ml) of RPMI1640 medium was added thereto, and the mixture was centrifuged (1500 rpm, 5 minutes) to remove the supernatant. The resultant was added to a 10% FCS-RPMI1640 medium (HAT medium) containing hypoxanthine (100 µM), aminopterin (0.4 µM) and thymidine (10 µM) wherein the spleen cells were contained in 1 x 10⁶/ml.

### (3) Selection of hybridoma

The cell-floating solution prepared in the above (2) was poured into 96 well microplates (5 plates) in an amount 200 µl per each well, and cultured in a CO₂ incubator (5% CO₂) at 37°C. After one week, it was confirmed that only the hybridoma grew and formed colonies.

### (4) Detection of antibody

After confirming the growth of the hybridoma, the antibody was detected by using the culture supernatant as follows.

The detection of antibody was done based on the inhibitory effect of CD-28 positive dependent CD8⁺ cell strains, YT2C2 (available from Juntendo University, School of Medicine, the Department of Immunology) against JY cell. The inhibitory effects were carried out by the following method.

### (i) Preparation of effector cells

Cells were recovered from the culture broth of CD28 positive cytotoxic cell strain, YT2C2, and there was prepared a cell suspension (25 ml) in a 10% FCS-RPMI1640 medium in a concentration of 4 x 10⁶ cells/ml.

### (ii) Preparation of target cells

The JY cells were recovered and suspended in FCS in 1 x 10⁷ cells/ml. Thereto was added Na₂⁵¹CrO₄ (sodium chromate) (manufactured by Daiichi Kagaku) 1.85 MBq (50 µl) which had isotope activity of ⁵¹Cr, and the mixture was incubated at 37°C for one hour by which the ⁵¹Cr was taken into the cells. The resulting cells were washed by centrifuge with a 10% FCS-RPMI1640 three times, and then, there was prepared a suspension (25 ml) in a 10% FCS-RPMI1640 in a concentration of 2 x 10⁵ cells/ml.

### (iii) Cytotoxic reaction

The effector YT2C2 was added to a 96 well U-type plate (manufactured by Corning) in an amount of 50 µl/well and thereto was further added ⁵¹Cr-labelled JY in an amount of 50 µl/well. Thereto were added an anti-human B7 antibody (Becton-Dickinson) (20 µg/ml) in a 10% FCS-RPMI1640 and the culture supernatant of the grown hybridoma (50 µl/well). The contents of each well were well mixed, and thereafter, it was centrifuged at 1,000 rpm for one minute, and then reacted at 37°C for 4 hours. After 4 hours reaction, the ⁵¹Cr released in the culture supernatant was measured by the following method.

That is, the supernatant was recovered with a culture supernatant recovering strip (manufactured by Scatoron), and the amount of ⁵¹Cr was measured with a γ-ray counter (manufactured by Pharmacia) and the data were converted into the cytotoxicity. On the basis of the resultants, there was selected one well which contained a hybridoma being capable of secreting an antibody having an inhibitory activity against cytotoxicity of YT2C2 to JY.

### (5) Cloning

The hybridoma grown in well as prepared in the above (4) was cloned by limiting dilution method. The resultant was diluted with a 10% FCS-RPMI1640 medium so as to make the final cell concentration to one/well and each 200 µl thereof were aliquoted into 96 well plate and cultured at 37°C under 5% CO₂. After about one week, growth of hybridoma was observed. When the colonies became in a certain scale, the antibody was again detected in the same manner as described in the above (4). The above procedure was repeated twice to select a clone which could stably produce an antibody to the molecule other than B7/BB1 binding to CD28 and CTLA-4, i.e. an antibody to the B70 molecule. Thus, YT2C2 inhibited JY. While anti-CD28 antibody completely blocked the cytotoxicity, the anti-B7 antibody could only partially block the cytotoxicity. The antibody obtained above could completely block the cytotoxicity, and hence, it may be assumed that it will be an antibody to CD28-binding molecule other than B7. This was confirmed by immunoprecipitation as described in (9) hereinafter. This hybridoma was designated as "IT-2" and has been originally deposited as a domestic microorganism deposit (FERM P-13823, deposited on August 26, 1993) at the "National Institute of Bioscience and Human-Technology, Agency of Industrial Science and Technology', Tsukuba-shi, Ibaraki-ken, Japan and converted into an international one (FERM BP-4780) under the Budapest Treaty on August 12, 1994.

### (6) Determination of subclass of the antibody

The subclass of the antibody produced by said hybridoma was determined by an enzyme linked immunosolvent assay (ELISA). That is, the anti-monoclonal antibody on the culture supernatant of the hybridoma was reacted with an anti-mouse antibody [anti-IgG2a, IgG₁, IgG2b, IgG3, IgM] and an anti-mouse L chain antibody [anti-κ, -λ] (MAB-isotyping kit, Pharmingen). As a result, coloring was observed only in the well which contained IgG₁, κ, and hence, it was found that the hybridoma can produce a monoclonal antibody of IgG₁, κ chain.

### (7) Inhibitory effects of IT-2 antibody against cytotoxicity

The antibody produced by the hybridoma thus obtained was purified from the mouse ascites. The inhibitory effect of the antibody against the cytotoxicity of YT2C2 cell was tested by using the purified antibody. The test was made by the same antibody detection method as described in the above (4).

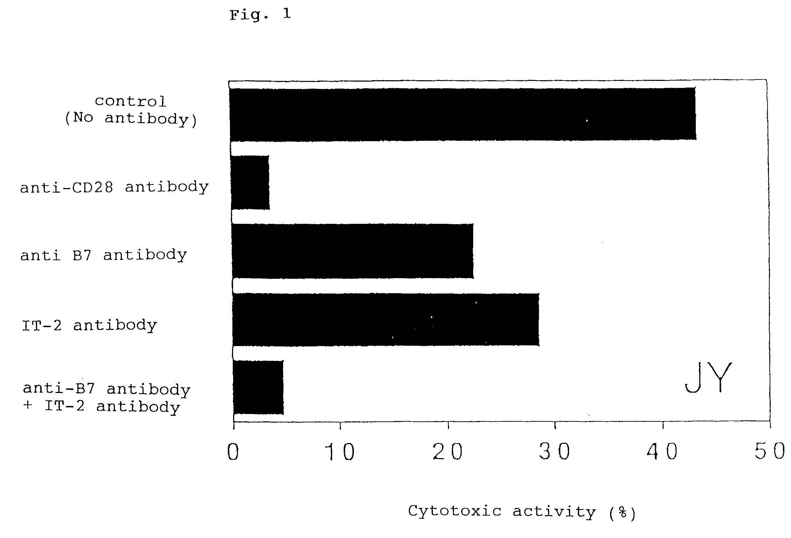

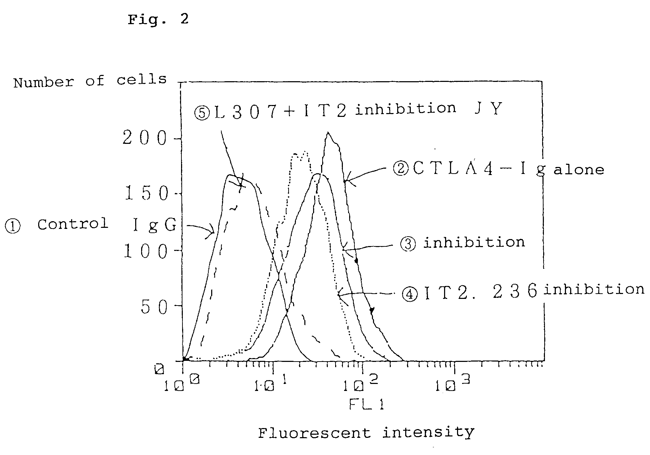

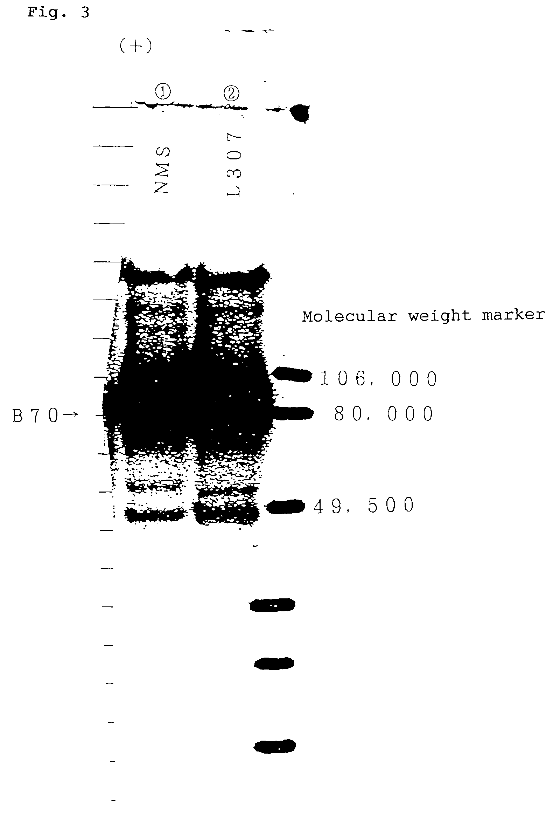

An effector cell, YT2C2 (8 x 10⁶ cells/ml) was aliquoted into 96 well U type plate in an amount of 50 µl/well, and thereto was added ⁵¹Cr-labelled JY cell (2 x 10⁵ cells/ml) in an amount of 50 µl/well. To the well were further added the purified antibody, i.e. anti-B7 antibody (Becton-Dickinson), anti-CD28 antibody (Becton-Dickinson) and IT-2 antibody (each 20 µg/ml) in each amount of 50 µl/well. After reacting for 4 hours, ⁵¹Cr released in the culture supernatant was counted with a γ-ray counter. The obtained data was converted into the cytotoxicity-inhibitory activity, and each data were compared. The results are shown in the attached Fig. 1. As is clear from Fig. 1, the cytotoxicity of YT2C2 cell was completely inhibited in the presence of anti-B7 antibody and IT-2 antibody in the same degree as in the case of anti-CD28 antibody alone.

### (8) Inhibitory effects of IT-2 antibody against the binding of CTLA-4 to B7/BB1 and B70

The inhibitory activity of IT-2 antibody against the binding of CD28 and CTLA-4 molecule to B70 molecule was tested as follows.

By using an extracellular domain of CTLA-4 which has higher affinity to B7/BB1 than CD28 and a CTLA-4-Ig chimera molecule fused with Fc of human IgG₁ (available from Juntendo University, School of Medicine, the Department of Immunology), the inhibitory activity of IT-2 antibody and anti-B7 antibody against the binding of JY and CTLA-4-Ig was tested.

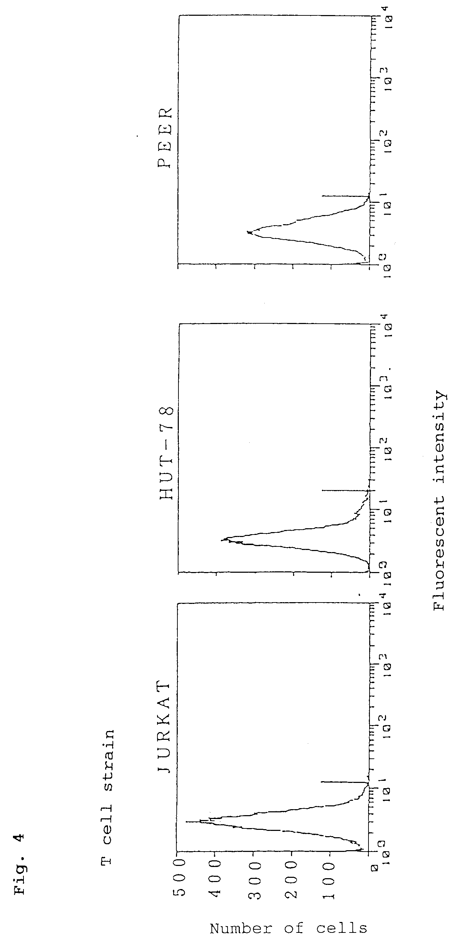

Firstly, JY cells (1 x 10⁶ cells/100 µl) were entered into Fisher's tube, and thereto were added IT-2 antibody (1 µg), or B7 antibody (1 µg), or a mixture of IT-2 antibody (1 µg) and anti-B7 antibody (1 µg), and they were reacted at 4°C for 30 minutes. The reaction mixture was washed twice with PBS with centrifugation, and thereto was added CTLA-4-Ig molecule (1 µg), and they were further reacted at 4°C for 30 minutes. After washing twice with centrifugation, anti-human IgG.Fd-FITC antibody (secondary antibody, Cappel) was reacted thereto. After the reaction, the reaction mixture was analyzed by flow cytometry (FACS can; manufactured by Becton-Dickinson). The results are shown in the attached Fig. 2. As is clear from Fig. 2, IT-2 antibody inhibited completely the binding of JY and CTLA-4-Ig in the copresence of anti-B7 antibody. In Fig. 2, the symbols have the following meanings.
L307 = anti-B7 antibody
IT2, 236 = IT-2 antibody
1: Only Control IgG₁
2: Reaction of CTLA-4-Ig with JY
3: Inhibition of the reaction of CTLA-4-Ig with JY by anti-B7 antibody
4: Inhibition of the reaction of CTLA-4-Ig with JY by IT-2 antibody
5: Inhibition of the reaction of CTLA-4-Ig with JY by anti-B7 antibody and IT-2 antibody

### (9) Immunoprecipitation (Immunoprecipitation of B70 molecule from JY cells by IT-2 antibody)

JY cells (2 x 10⁷ cells/ml) were cultured in a 75 cm² culture flask which contained a 10% FCS-RPMI1640 medium. The JY cells were recovered by centrifuging at 1,500 rpm for 5 minutes and washed once with PBS with centrifugation and further washed three times with HBSS (Hank's buffer) with centrifugation.

### (i) Biotinylation of the cells

After removing the supernatant of the above culture broth by aspiration with suction, the resulting pellet was suspended in a 0.1M Hepes buffer (pH 8.0, 2 ml), and thereto was added an NHS-biotin (10 mg/ml) solution (20 µl), and the mixture was reacted with a rotator at room temperature for 40 minutes. After the reaction, the reaction mixture was cooled to 4°C and washed three times with RPMI1640 solution with centrifugation.

### (ii) Dissolution of the cells

To the pellet of the biotinylated cells was added a lytic buffer [50 mM Tris-HCl, 150 mM NaCl, 1% Triton X-100, 50 mM iodoacetamide, 2 mM MgCl₂, 2mM CaCl₂, 0.1% sodium azide, 10 µg/ml soybean trypsin inhibitor, 1 U/ml aprotinin, 1mM PMSF (phenylmethylsulfonyl fluoride), 1 µg/ml leupeptine] (400 µl), and the mixture was well stirred and then allowed to stand on ice for 30 minutes. Thereafter, the reaction mixture was centrifuged at 15,000 rpm for 10 minutes, and the supernatant was recovered. The supernatant was divided into two tubes (each content, 200 µl).

### (iii) Preclear

To the above tubes were added a CNBr activated Sepharose gel (manufactured by Pharmacia) bond with normal mouse serum (200 µl) and a CNBr activated Sepharose gel (manufactured by Pharmacia) bond with an anti-B7 antibody (200 µl), respectively. Each the mixture was reacted at 4°C overnight and then centrifuged at 12,000 rpm for one minute to precipitate the gel, and the supernatant was recovered. (The binding of the normal mouse serum and the anti-B7 antibody to the CNBr activated Sepharose gel was done in accordance with the manual of Pharmacia)

### (iv) Immunoprecipitation

Each supernatant thus recovered was added to Sepharose gel (100 µl) bond with an antibody produced by hybridoma IT-2 and the mixture was reacted at room temperature for 2 hours. The reaction mixture was washed three times with a washing buffer (50 mM Tris-HCl, pH8.3, 0.6 M sodium chloride, 0.5% NP-40, 0.1% sodium azide). To the resulting gel was added a sample buffer containing 2% SDS (sodium dodecylsulfate) [10% glycerol, 5% 2-mercaptoethanol, 2.3% SDS, 0.625 M Tris-HCl, pH 6.8, BPB (bromophenol blue) 1 mg/100 ml] (20 µl), and the mixture was boiled for 5 minutes. The reaction mixture was subjected to electrophoresis with 4 - 20% SDS-PAGE gel (manufactured by Daiichi Kayaku). After the electrophoresis, it was transferred to a nitrocellulose membrane with a transblotting system (manufactured by Bio-Rad) in a usual manner.

### (v) Development reaction

The nitrocellulose membrane was subjected to blocking by dipping in a 1% BSA (bovine serum albumin)-PBS for one hour. Thereafter, it was dipped in ABC solution (avidin-biotinylated peroxidase mixture (manufactured by Vector) and reacted for 30 minutes. The resultant was washed three times with 0.05% Tween 20/PBS (incubation for 10 minutes, three times). It was reacted with a development kit of ECL Western Blotting Detection System (Amersham) and an X-ray film was exposed thereto, by which the protein to be reacted with the hybridoma IT-2 was detected. The results are shown in the attached Fig. 3. As is clear from the figure, about 70 kd of protein were precipitated, by which it was confirmed that the antibody of this invention is a novel antibody to a CD28 binding protein other than B7/BB1. This protein was designated as "B70". In Figure 3, each signal has the following meaning.
1: Preclear with normal mouse serum
2: Preclear with anti-B7 antibody

### (12) Staining of various cells with FACS

By using the antibody produced by the obtained IT-2 hybridoma, there was determined the distribution of cells wherein CD28 and CTLA-4 binding molecules other than B7/BB1 were expressed. That is, various cell strains, peripheral blood lymphocytes and tonsillar lymphocytes suffered from chronic tonsillitis were analyzed with FACS (flow cytometery).

Each cells were prepared in a solution in PBS (cell content, 1 x 10⁶ cells/ml) and entered into Fisher tube. Thereto was added IT-2 antibody (1 µg), and the mixture was reacted on ice for 30 minutes. The reaction mixture was washed with PBS with centrifugation (3,000 rpm, for one minute, twice) and thereto was further added FITC-anti-mouse Ig's (manufactured by Olympus) (100-folds dilution) (100 µl), and the mixture was further reacted on ice for 20 minutes. After the reaction, the reaction mixture was washed twice with PBS with centrifugation and then suspended in PBS (200 µl), and then the cell number was measured with FACS can.

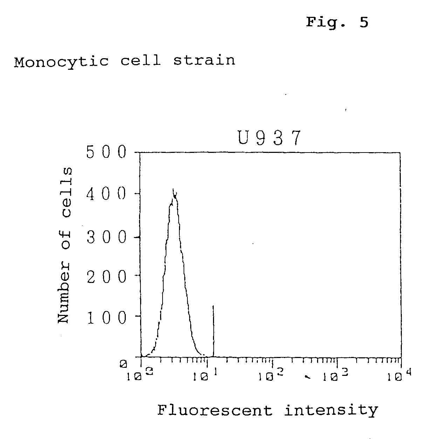

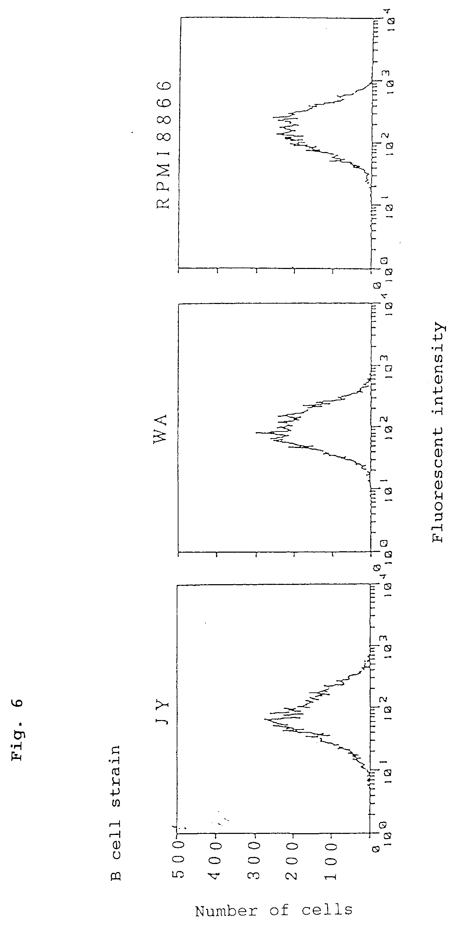

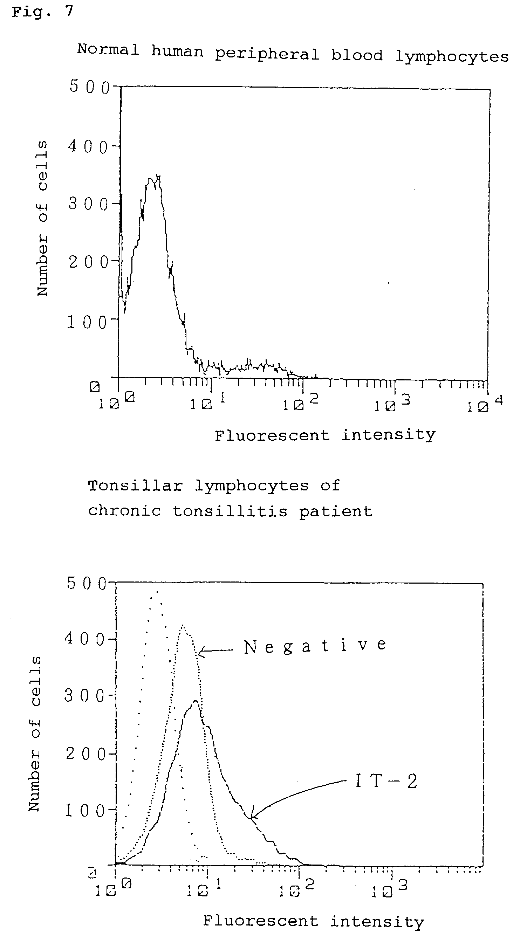

As the result, as shown in the attached Fig. 4, 5 and 6, there was observed no expression in case of T cell strains such as MOLT-4 (ATCC CRL 1582), PEER (available from Juntendo University, School of Medicine, the Department of Immunology), HUT-78 (ATCC TIB 161), JURKAT (available from Juntendo University, School of Medicine, the Department of Immunology), etc., and monocytic cell strains such as THP-1 (ATCC TIB 202), U937 (ATCC CRL 1593), etc. , but on the contrary, there was observed strong expression in case of EBV-transformed B cells such as JY, WA (both available from Juntendo University, School of Medicine, the Department of Immunology), RPMI8866 (available from Juntendo University, School of Medicine, the Department of Immunology), etc. Besides, as is shown in Fig. 7, normal human's peripheral blood lymphocytes did not show any expression, but there was observed strong expression in case of tonsillar lymphocytes of a patient suffered from tonsillitis.

Based on the above results, it was confirmed that the molecule recognized by the antibody produced by IT-2 hybridoma can specifically express in activated B cells.

As is clear from descriptions above, the antibody of this invention is useful for analysis of interaction between cells such as T-B cells in the field of basic study of immunology and further is useful for the diagnosis of autoimmune diseases, chronic tonsillitis to which an activated B cell participates.

Moreover, since the antibody of this invention can inhibit the co-stimulatory signal of the cells via CD28 together with anti-B7 antibody, it will be able to induce an antigen-specific tolerance, and hence, it will also be useful as immunosuppressor in transplantation. The bispecific antibody of the anti-B7 antibody will be more effective.

## Claims

1. A monoclonal antibody against a molecule which is expressed specifically in an activated B cell, or its functional fragment.

2. The monoclonal antibody or its functional fragment according to claim 1, wherein the molecule to be expressed specifically in an activated B cell is a protein having a molecular weight of about 70 kd.

3. The monoclonal antibody or its functional fragment according to claim 1, wherein the molecule to be expressed specifically in an activated B cell is B70 molecule which is a molecule being capable of binding to CD28 or CTLA-4 and is distinguished from B7/BB1 molecule.

4. The monoclonal antibody or its functional fragment according to claim 3, which has an inhibitory effect the binding of B70 to CD28 or CTLA-4.

5. The monoclonal antibody or its functional fragment according to claim 4, which has an inhibitory effect on CD28-dependent T cell lysis of B70 presenting cells.

6. A method for the production of a monoclonal antibody which is specifically reactive to B70 molecule, which comprises:
(i) immunizing a rodent with a human B cell strain (JY);
(ii) taking out the spleen from the immunized rodent and preparing a suspension of spleen cells;
(iii) mixing the spleen cell suspension with mouse myeloma cells in the presence of a fusion-promoting agent to effect the fusion of both cells;
(iv) culturing the fused cells in a medium which does not support unfused myeloma cells to select a hybridoma formed by fusion of the antibody-producing cells and myeloma cells;
(v) confirming the presence of anti-B70 antibody in the supernatant of each culture well containing the hybridoma with the indication of the complete inhibitory effect on CD28-positive cell strain YT2C2 (NK cell) lysis of JY (B cell strain);
(vi) selecting the hybridoma being capable of producing the desired antibody, and obtaining a single clone by a limiting dilution method; and
(vii) recovering the antibody from the culture supernatant of the hybridoma of single clone.

7. The method according to claim 6, wherein the rodent is Balb/c mouse and the mouse myeloma cell is P3U1.

8. A hybridoma being capable of producing a monoclonal antibody which specifically reacts with B70 molecule on the surface of activated B cells.

9. Pharmaceutical composition, comprising a pharmaceutically active amount of the monoclonal antibody according to claim 1 and at least one pharmaceutically acceptable carrier or excipient.

10. Use of the monoclonal antibody according to claim 1 for the preparation of a pharmaceutical composition for suppressing immunoreactions in transplantation.

11. Use of the monoclonal antibody according to claim 1 for the preparation of a diagnostic for diseases selected from autoimmune disease and chronic tonsillitis.
